## Europäisches Patentamt
## European Patent Office
## Office européen des brevets

(19)

(11) **EP 1 315 528 B1**

(12) **EUROPÄISCHE PATENTSCHRIFT**

(45) Veröffentlichungstag und Bekanntmachung des Hinweises auf die Patenterteilung:
**28.04.2004 Patentblatt 2004/18**

(21) Anmeldenummer: **01947340.4**

(22) Anmeldetag: **05.06.2001**

(51) Int Cl.$^7$: **A61L 15/60**, C08L 101/14

(86) Internationale Anmeldenummer:
**PCT/EP2001/006362**

(87) Internationale Veröffentlichungsnummer:
**WO 2002/020068 (14.03.2002 Gazette 2002/11)**

(54) **PULVERFORMIGE, VERNETZTE, WÄSSRIGE FLÜSSIGKEITEN SOWIE BLUT ABSORBIERENDE POLYMERE**

PULVERULENT, CROSS-LINKED POLYMERS, CAPABLE OF ABSORBING AQUEOUS LIQUIDS AND BLOOD

POLYMERES RETICULES PULVERULENTS ABSORBANT DES LIQUIDES AQUEUX ET DU SANG

(84) Benannte Vertragsstaaten:
**AT BE CH CY DE DK ES FI FR GB GR IE IT LI LU MC NL PT SE TR**

(30) Priorität: **04.09.2000 DE 10043706**

(43) Veröffentlichungstag der Anmeldung:
**04.06.2003 Patentblatt 2003/23**

(73) Patentinhaber: **STOCKHAUSEN GmbH & Co. KG**
**47805 Krefeld (DE)**

(72) Erfinder:
• **MERTENS, Richard**
  **47803 Krefeld (DE)**
• **HARREN, Jörg**
  **47807 Krefeld (DE)**

(74) Vertreter: **Herzog, Martin**
  **Kahlhöfer . Neumann . Herzog . Fiesser,**
  **Karlstrasse 76**
  **40210 Düsseldorf (DE)**

(56) Entgegenhaltungen:
**EP-A- 0 233 067     EP-A- 0 574 260**
**EP-A- 0 668 080     EP-A- 0 712 659**
**EP-A- 0 837 076     WO-A-98/49221**
**DE-A- 19 846 412**

**Beschreibung**

Die Erfindung betrifft pulverförmige, vernetzte, Wasser, wässrige Flüssigkeiten sowie Blut absorbierende Polymere (Superabsorber), mit verbesserten Eigenschaften insbesondere mit einer verbesserten Retention und einem verbesserten Rückhaltevermögen von Flüssigkeiten unter Druck und einer verbesserten Fähigkeit Flüssigkeiten zu transportieren, deren Herstellung und deren Verwendung als Absorptionsmitttel in Hygieneartikeln und in technischen Bereichen

Superabsorber sind wasserunlösliche, vernetzte Polymere, die in der Lage sind, unter Quellung und Ausbildung von Hydrogelen große Mengen an wässrigen Flüssigkeiten und Körperflüssigkeiten, wie z. B. Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikel, wie z. B. Babywindeln, Inkontinenzprodukten oder Damenbinden.

Bei den gegenwärtig kommerziell verfügbaren Superabsorbern handelt es sich im Wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht die zunehmende Tendenz, die Sanitärartikel wie Babywindeln, Inkontinenzprodukte und Damenbinden immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozeß läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozeß, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstärke zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzem und Comonomeren, wie in Patentschrift DE 196 46 484 beschrieben, vermag die Permeabilitätseigenschaften zwar zu verbessern, nicht aber auf ein Niveau, das beispielsweise den Einbau einer gegebenenfalls nur aus Superabsorbern bestehende Schicht in eine Windelkonstruktion erlaubt.

Weiterhin können Methoden zur oberflächlichen Nachvernetzung der Polymerpartikel zur Anwendung kommen. Bei der sog. Nachvernetzung werden die Carboxylgruppen der Polymermoleküle an der Oberfläche der Superabsorberpartikel mit verschiedenen Nachvernetzungsmitteln, die mit mindestens zwei der oberflächennahen Carboxylgruppen reagieren können, zur Reaktion gebracht. Neben der Erhöhung der Gelstärke wird insbesondere die Fähigkeit zur Flüssigkeitsaufnahme unter Druck stark verbessert, da das bekannte Phänomen des Gel-Blocking unterdrückt wird, bei dem angequollene Polymerteilchen verkleben und dadurch eine weitere Flüssigkeitsaufnahme verhindert wird.

Die Oberflächenbehandlung von flüssigkeitsabsorbierenden Harzen ist bereits bekannt. Zur Verbesserung der Dispergierbarkeit wird eine ionische Komplexierung der oberflächennahen Carboxylgruppen mit polyvalenten Metallkationen in der US 4,043,952 vorgeschlagen. Die Behandlung erfolgt mit Salzen mehrwertiger Metalle, die in organischen, ggf. Wasser enthaltende Solventien, (Alkohole und andere organische Solventien), dispergiert sind.

Eine Nachbehandlung von Superabsorberpolymeren mit reaktionsfähigen, oberflächenvernetzenden Verbindungen (Alkylencarbonate) zur Erhöhung der Flüssigkeitsaufnahmefähigkeit unter Druck wird in DE-A-40 20 780 beschrieben.

Eine Oberflächennachvernetzung von superabsorbierenden Polymeren mit polyfunktionellem Vernetzer wie polyvalenten Metallverbindungen in Gegenwart von inertem, anorganischem Pulver wie $SiO_2$ zur Verbesserung der Absorptionseigenschaften und zur Erzeugung eines nichtklebenden Gels der Polymerteilchen wird in DE-A-35 03 458 beschrieben.

Gemäß der Lehre von EP-A-0 574 260 erhält man superabsorbierende Polymere mit einem niedrigen Restmonomergehalt, der sich auch bei einer Oberflächenvernetzung nicht entscheidend verändert, wenn man bei der Polymerisation bestimmte Bedingungen einhält und die Nachvernetzung mit üblichen mehrfunktionellen Vernetzern, wie Polyolen, Alkylencarbonaten, polyvalenten Metallsalzen bei üblichen Bedingungen durchführt. Die nachvernetzten Polymeren zeigen eine gute Absorption ohne Druckanwendung.

Gemäß EP-A-0 889 063 können superabsorbierende Polymere, die bereits vorzugsweise oberflächenvernetzt sind, durch Nachbehandlung mit einer Verbindung des Titans oder Zirkons und eine diese Metallverbindungen chelatierende Verbindung gegen radikalischen Abbau durch Körperflüssigkeiten, insbesondere L-Askorbinsäure, ausgerüstet werden.

Die EP 0 233 067 beschreibt wasserabsorbierende, an der Oberfläche vernetzte Harze, die durch Reaktion von einem superabsorbierenden Polymerpulver mit 1 - 40 Gew.%, bezogen auf das Polymerpulver, einer Aluminiumverbindung erhalten werden. Als Behandlungslösung findet eine Mischung aus Wasser und Diolen Verwendung, die den Einsatz von niederen Alkoholen als Lösemittel überflüssig machen soll. Es werden bevorzugt 100 Gew.Teile Vernetzerlösung auf 100 bis 300 Gew.Teile Absorber aufgebracht. Die dem Reaktionsmedium Wasser zugefügten Diole (z. B. Polyethylenglycol 400 und 2000, 1,3-Butandiol oder 1,5-Pentandiol) dienen u. a. auch dazu, ein Verklumpen des Superabsorbers bei der Behandlung mit den hier verwendeten großen Mengen an wässriger Behandlungslösung zu verhindern. Das Lösemittel wird in einer anschließenden Trocknung bei 100°C entfernt. Die so behandelten Polymere weisen ein nicht ausreichendes Eigenschaftsniveau auf, wobei eine Verbesserung der Absorptionsfähigkeit unter Druck nicht erreicht wird. Außerdem ist eine Behandlung mit großen Mengen Behandlungslösung bei modernen, kontinuierlich arbeitenden Verfahren nicht ökonomisch durchführbar.

In der WO 96/05234 wird ein Verfahren zur Behandlung von superabsorbierenden Polymeren beschrieben, gemäß dem die Oberfläche der mindestens 10 Gew.% Wasser enthaltenden Absorberteilchen mit einer vernetzten Schicht erhalten durch eine Reaktion von einem reaktiven, hydrophilen Polymeren oder einer reaktiven metallorganischen Verbindung mit einem mindestens bifunktionellen Vernetzer bei Temperaturen unter 100°C ausgerüstet wurde. Metallsalze werden nicht aufgeführt. Die zum Einsatz kommenden Metallverbindungen müssen mit den funktionellen Gruppen des Vernetzers reagieren können. Als Metallverbindungen werden daher metallorganische Verbindungen empfohlen, die zu der Vernetzerverbindung im Gewichtsverhältnis 0,1 bis 30 vorliegen sollen. Die erhaltenen Polymerisate sollen ein ausgewogenens Verhältnis von Absorption, Gelfestigkeit und Permeabilität aufweisen, wobei die angegebenen Meßwerte unter weniger kritischen Bedingungen ermittelt werden. So werden beispielsweise die Absorption und die Permeabilität ohne jegliche Druckbelastung bestimmt. Nachteilig ist bei diesem bekannten Verfahren die Verwendung von Lösemitteln und toxisch bedenklichen Vemetzungsreagentien wie z.B. den als bevorzugt genannten Polyiminen, alkoxylierten Silan- bzw. Titan-Verbindungen und Epoxiden.

Durch eine entsprechende Behandlung von kommerziell erhältlichen Superabsorberpolymeren mit Aminopolymeren in organischen Lösungsmitteln wird gemäß der Lehre von WO 95/22356 und WO 97/12575 eine Verbesserung der Permeabilitäts- und Flüssigkeitstransporteigenschaften erreicht. Der gravierende Nachteil des hier beschriebenen Verfahrens liegt neben der Verwendung von toxikologisch bedenklichen Polyaminen und Polyiminen in dem Einsatz großer Mengen organischer Lösungsmittel, die für die Behandlung der Polymere notwendig sind. Der damit verbundene Sicherheitsaspekt und Kostenaufwand schließt eine großtechnische Produktion aus. Neben der toxikologischen Bedenklichkeit dieser Behandlungsmittel ist weiterhin zu berücksichtigen, daß sie unter den hohen Nachvernetzungstemperaturen auch zur Zersetzung neigen, was sich u.a. in einer Gelbfärbung der Absorberpartikel äußert.

Zur Herstellung von Wasser absorbierenden Polymeren mit besserer Abriebfestigkeit wird in der japanischen Offenlegungsschrift JP-A-09124879 die Oberflächennachvemetzung mit polyfunktionellen Vernetzer gelehrt, wobei der Wassergehalt der Polymerteilchen nach der Oberflächenvernetzung wieder auf 3 - 9 Gew.% eingestellt wird und diese Wassermenge anorganische Verbindungen wie Metallsalze enthalten kann.

Superabsorbierende Polymere, die gemäß WO 98/48857 in Teilchenform mit polyvalenten Metallsalzen durch trockenes Mischen in Kontakt gebracht und anschließend mit einer bestimmten Menge eines flüssigen Bindemittels, wie Wasser oder Polyole, versehen werden, sollen ein verbessertes Gelblocking bei der Absorption von wässrigen Flüssigkeiten aufweisen. Vor dieser Behandlung können die Polymerteilchen einer Oberflächennachvemetzung unterworfen werden.

Zur Minimierung der Agglomerationsneigung von superabsorbierenden, nachvernetzten Polymerteilchen durch elektrostatische Aufladung wird in WO 98/49221 empfohlen, die Polymerteilchen mit einer wäßrigen Additivlösung bis zu 10 Gew.% Wasser wieder zu befeuchten. Diese wäßrigen Lösungen können mono- oder polyvalente Ionen oder propoxylierte Polyole enthalten. Es ist auch möglich, die Polymerteilchen bereits vor der Oberflächennachbehandlung mit der wäßrigen Additivlösung in Kontakt zu bringen, wodurch eine gleichmäßigere Verteilung der Oberflächennachbehandlungsmittel erreicht werden soll.

Einen Hinweis darauf, daß unter Beibehaltung einer hohen Retentionskapazität und Aufnahmefähigkeit von Flüssigkeit unter Druck bei der Nachvemetzungsstufe ebenfalls die Permeabilitätseigenschaften drastisch gesteigert werden können, ist aus dem vorstehend beschriebenen Stand der Technik nicht zu erkennen.

Aufgabe der vorliegenden Erfindung war es daher, superabsorbierende Polymere bereitzustellen, die eine verbesserte Eigenschaftkombination aufweisen, insbesondere nicht nur eine hohe Aufnahmekapazität unter Druck, sondern auch die üblicherweise gegenläufigen Eigenschaften eines hohen Retentionsvermögens und einer guten Permeabilität in sich vereinigen, d. h. ein Niveau der Eigenschaftskombination aufweisen, bei dem neben einem Retentionswert von $\geq$ 25 g/g mindestens ein SFC-Wert von mindestens $45 \cdot 10^{-7}$, vorzugsweise von mindestens $50 \cdot 10^{-7} cm^3$ s /g vorliegt. Insbesondere lag die Aufgabe darin, superabsorbierende Polymere zur Verfügung zu stellen, die sich vor allem für die Verwendung in sehr dünnen Windelkonstruktionen mit sehr hohem Superabsorberanteil eignen. Für diesen Fall sind insbesondere Polymere mit Retentionswerten von $\geq$ 25 g/g und Permeabilitätswerte von SFC > $70 \cdot 10^{-7} cm^3$ s /g erforderlich.

Eine weitere Aufgabe der Erfindung war es, Herstellungsverfahren für solche superabsorbierenden Polymeren zu finden, die einfach, ökonomisch und sicher durchführbar sind, eine gleichmäßige Produktqualität liefern und bei denen insbesondere niedrige Lösungsmittelmengen verwendet und organische Lösungsmittel nach Möglichkeit vermieden werden. Darüber hinaus sollen die Verfahren ohne die Verwendung toxikologisch bedenklicher Substanzen durchführbar sein.

Die erfindungsgemäße Aufgabe wird durch die Bereitstellung eines pulverformigen, an der Oberfläche nachvernetzten, Wasser, wäßrige oder seröse Flüssigkeiten sowie Blut absorbierenden Polymerisates, aufgebaut aus

a) 55-99,9 Gew% polymerisierten, ethylenisch ungesättigten, säuregruppenenthaltenden Monomeren, die zu mindestens 25 Mol% neutralisiert sind,

b) 0-40 Gew% polymerisierten, ethylenisch ungesättigten, mit a) copolymerisierbaren Monomeren,

c) 0,1 - 5,0 Gew% eines oder mehrerer einpolymerisierter Vernetzer,

d) 0-30 Gew% eines wasserlöslichen Polymeren
wobei die Summe der Gewichtsmengen a) bis d) 100 Gew.% beträgt, **dadurch gekennzeichnet,** daß das Polymerisat mit

e) 0,01 bis 5 Gew.%, bezogen auf das Polymerisat, wenigstens eines Polyols als Oberflächennachvernetzermittel in einer wässrigen Lösung und mit

f) 0,001 - 1,0 Gew%, bezogen auf das Polymerisat, eines Kations in Form eines in einer wäßrigen Lösung gelösten Salzes beschichtet und auf eine Nachvernetzungstemperatur von >150 bis 250 °C erhitzt worden ist, wobei das Gewichtsverhältnis von Salz zu Polyol im Bereich von 1:0,8 bis 1:4 liegt und Gesamtmenge der wäßrigen Lösungen 0,5 bis 10 Gew.%, bezogen auf das Polymerisat, betragen hat, mit Ausnahme von vernetzter, teilneutralisierter Polyacrylsäure, die mit $Al_2(SO_4)_3$ und Glycerin im Gewichtsverhältnis 1:1 bzw. mit $Al_2(SO_4)_3 \cdot 16 H_2O$ und Polyethylenglykol im Gewichtsverhältnis 1:1,8 bzw. mit $Al_2(SO_4)_3 \cdot 14 H_2O$ zu Ethylenglykol im Gewichtsverhältnis 1:2 bzw. mit $Al_2(SO_4)_3 \cdot 18 H_2O$ zu Ethylenglykol im Gewichtsverhältnis 1:2 bzw. $Al_2(SO_4)_3 \cdot 18 H_2O$ zu Propylenglykol im Gewichtsverhältnis 1:1,6 behandelt worden ist, gelöst.

Überraschenderweise ergibt sich nämlich durch die Beschichtung eines teilchenförmigen absorbierenden Polymerisats mit einer wässrigen Lösung eines Polyols, das mit den oberflächennahen Molekülgruppen, vorzugsweise mit den Carboxylgruppen, in Anwesenheit eines Kations eines wasserlöslichen Salzes unter Erhitzung auf > 150 bis 250 °C reagiert hat, ein superabsorbierendes Polymerisat mit einer signifikanten Verbesserung der Permeabilitätseigenschaften bei sehr gutem Retentionsvermögen, wenn das wasserlösliche Salz zu dem Polyol in einem bestimmten Gewichtsverhältnis vorliegt und die zugeführte Wassermenge in den erfindungsgemäßen Grenzen liegt.

Völlig unerwartet führt die Behandlung mit wässriger Lösung der erfindungsgemäßen Kombination von Nachvernetzer-Komponenten zum erwünschten Ergebnis, nämlich Polymerisate mit einem hohen Retentionsvermögen auch unter Druck bei gleichzeitig ausgezeichneten Permeabilitätseigenschaften zu erhalten. Eine aufeinander folgende separate Anwendung der wässrigen Lösung des organischen Nachvernetzungsmittels und der wässrigen Salzlösung mit jeweiligem Erhitzen führt nicht zu einer vergleichbar guten Produktcharakteristik.

Die alleinige Verwendung eines Polyols als organisches Nachvernetzungsmittel in wässriger Lösung führt zwar zu Produkten mit hoher Retentionskapazität, hoher Gelstärke und hohem Aufnahmevermögen unter Druck. Eine signifikante Steigerung der Permeabilität im gequollenen Zustand kann allerdings nur durch einen entsprechend höheren Grad der Vernetzung der Polymere bei der Polymerisation, bzw. einer stärkeren Nachvernetzung (erhöhte Mengen Nachvernetzungsmittel oder drastischere Bedingungen) und dem damit verbundenen Verlust an Retentionskapazität erreicht werden.

Die alleinige Nachvernetzung mit Kationen hoher positiver Ladungsdichte führt ebenfalls nicht zu Polymerisaten mit der erwünschten Eigenschaftskombination. Insbesondere lassen sich keine befriedigenden Werte bei der Flüssigkeitsaufnahme unter Druck und keine guten Permeabilitätseigenschaften erreichen. Eine Verbesserung der Druckstabilität oder darüber hinaus der Flüssigkeitstransporteigenschaften im gequollenen Zustand wird nicht erreicht.

Die geforderten Eigenschaften können auch nicht durch eine kleine Menge Polyol und große Mengen Kation erreicht werden.

Erfindungsgemäß werden als organische Nachvemetzer-Komponente e) Polyole eingesetzt, die mit den oberflächennahen COOH-Gruppen des Polymerisats reagieren.

Vorzugsweise werden als Polyole aliphatische Polyhydroxyverbindungen, mit vorzugsweise einem Molekulargewicht von höchstens 250, wie $C_2$-$C_8$-Alkylendiole, wie z. B. Ethylenglykol, 1,3-Propandiol, 1,4-Butandiol, 1,5-Pentandiol, 1,6-Hexandiol, Dianhydrosorbit, $C_2$-$C_8$-Alkylentriole, wie z. B. Glycerin, Trimethylolpropan, höherfunktionelle Hydroxyverbindungen, wie z. B. Pentaerythrit und Zuckeralkohole, wie z. B. Sorbit sowie Di- und Polyalkylenglykole, wie z. B. Diethylenglykol, Dipropylenglykol, Triethylenglykol, Tetraethylenglykol, Tetrapropylenglykol, Polyethylenglykol, Polypropylenglykol, Polyglykole auf Basis von 2 oder mehr unterschiedlichen Alkoxiden, wie z. B. ein Polyglykol aus Ethylenoxid- und Propylenoxideinheiten, verwendet. Die organische Nachvemetzerkomponente bzw. deren Mischungen werden in Mengen von 0,01 - 5 Gew.%, bevorzugt 0,1 - 2,5 Gew.% und besonders bevorzugt von 0,5 bis 1,5 Gew.%, bezogen auf das zu vernetzende Polymerisat, eingesetzt.

Erfindungsgemäß werden als Komponente f) vorzugsweise die wässrigen Lösungen von von wasserlöslichen Salzen zur Vernetzung der oberflächennahen Carboxylatgruppen eingesetzt, deren Anionen Chloride, Bromide, Sulfate, Carbonate, Nitrate, Phosphate oder organische Anionen wie Acetate und Lactate sind. Die Kationen der Salze leiten sich vorzugsweise von ein- und mehrwertigen Kationen ab, die einwertigen insbesondere von Alkalimetallen, wie Kalium, Natrium, Lithium, wobei Lithium bevorzugt wird. Erfindungsgemäß verwendete zweiwertige Kationen leiten sich von Zink, Beryllium, Erdalkalimetallen wie Magnesium, Calzium, Strontium ab, wobei Magnesium bevorzugt wird. Weitere Beispiele für erfindungsgemäß einsetzbare höherwertige Kationen sind Kationen von Salzen von Aluminium, Eisen, Chrom, Mangan, Titan, Zirkonium und andere Übergangsmetalle sowie Doppelsalze solcher Kationen oder Mischungen der genannten Salze. Bevorzugt werden drei- und höherwertige Kationen und von diesen insbesondere wasserlösliche, anorganische Salze und von diesen Aluminiumsalze und Alaune und deren unterschiedliche Hydrate wie z.B. $AlCl_3$ x 6 $H_2O$, $NaAl(SO_4)_2$ x 12 $H_2O$, $KAl(SO)_4$ x 12 $H_2O$ oder $Al_2(SO_4)_3$ x 14 -18 $H_2O$ oder $Al(NO_3)_3$ x 9 $H_2O$ eingesetzt. Besonders bevorzugt werden $Al_2(SO_4)_3$ oder $Al(NO_3)_3$ und ihre Hydrate verwendet. Eingesetzt wird die Salzkomponente, berechnet auf das Kation, in Mengen von 0,001 - 1,0 Gew.%, bevorzugt 0,005 - 0,5 Gew.%, und besonders bevorzugt 0,01 - 0,2 Gew.%, bezogen auf das Polymerisat. Das bevorzugte Gewichtsverhältnis von wasserlöslichem Salz zum Nachvernetzungsrnittel beträgt vorzugsweise 1:1 bis 1:3,5, besonders bevorzugt 1:1,2 bis 1:2,5.

Das wasserabsorbierende Polymerisat, das erfindungsgemäß oberflächenvernetzt wird, wird u. a. durch Polymerisation von a) 55-99,9 Gew% eines einfach ethylenisch ungesättigten Monomeren mit Säuregruppen erhalten. Hierbei sind carboxylgruppenhaltige Monomere, wie z.B. Acrylsäure, Methacrylsäure, oder 2-Acrylamido-2-methylpropansulfonsäure oder Mischungen dieser Monomeren bevorzugt. Es ist bevorzugt, daß mindestens 50 Gew.% und besonders bevorzugt mindestens 75 Gew.% der Säuregruppen Carboxyl-Gruppen sind. Die Säuregruppen sind zu mindestens 25 Mol% neutralisiert , d.h. liegen als Natrium-, Kalium- oder Ammoniumsalze vor. Bevorzugt liegt der Neutralisationsgrad bei mindestens 50 Mol%. Besonders bevorzugt sind Polymerisate, die durch Polymerisation von Acrylsäure oder Methacrylsäure, deren Carboxylgruppen zu 50-80 Mol% neutralisiert ist, in Gegenwart von Vernetzern erhalten wurde.

Als weitere Monomere b) können für die Herstellung der erfindungsgemäßen absorbierenden Polymerisate 0-40 Gew% ethylenisch ungesättigte mit a) copolymerisierbarer Monomere, wie z. B. Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Über 40 Gew% dieser Monomerem können die Quellfähigkeit der Polymerisate verschlechtern.

Als Vernetzerkomponente c), die während der Polymerisation von a) und b) vorhanden ist, können alle Verbindungen verwendet werden, die mindestens zwei ethylenisch ungesättigte Doppelbindungen oder eine ethylenisch ungesättigte Doppelbindung und eine gegenüber Säuregruppen der Monomeren a) reaktive funktionelle Gruppe oder mehrere gegenüber Säuregruppen reaktive funktionelle Gruppen tragen. Beispielhaft seien genannt: aliphatische Amide wie z. B. das Methylenbisacryl- bzw. -methacrylamid oder Ethylenbisacrylamid, ferner aliphatische Ester von Polyolen oder alkoxylierten Polyolen mit ethylenisch ungesättigten Säuren, wie Di(meth)acrylate oder Tri(meth)acrylate, von Butandiol- oder Ethylenglykol, Polyglykolen, Trimethylolpropan, Di- und Triacrylatester des, vorzugsweise mit 1 bis 30 Mol Alkylenoxid oxalkylierten, vorzugsweise ethoxylierten Trimethylolpropans, Acrylat- und Methacrylatester von Glycerin und Pentaerythrit, sowie des mit vorzugsweise I bis 30 Mol Ethylenoxid oxethylierten Glycerins und Pentaerythrits, ferner Allylverbindungen wie Allyl(meth)acrylat, alkoxyliertes Allyl(meth)acrylat mit vorzugsweise 1 bis 30 Mol Ethylenoxid umgesetzt, Triallylcyanurat, Triallylisocyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie N-Methylolverbindungen von ungesättigten Amiden wie von Methacrylamid oder Acrylamid und die davon abgeleiteten Ether. Mischungen der genannten Vernetzer können ebenfalls eingesetzt werden.

Der Anteil an den vernetzenden Comonomeren liegt bei 0,1 bis 5 Gew%, bevorzugt bei 0,01 bis 3,0 Gew%, bezogen auf die Gesamtmenge der Monomeren.

Als wasserlösliche Polymere d) können in den erfindungsgemäßen absorbierenden Polymerisaten 0-30 Gew. % wasserlösliche Polymerisate, wie teil- oder vollverseifter Polyvinylacetat, Polyvinylpyrrolidon, Stärke oder Stärkederivate, Polyglycole oder Polyacrylsäuren, vorzugsweise einpolymerisiert, enthalten. Das Molekulargewicht dieser Polymeren ist unkritisch, solange sie wasserlöslich sind. Bevorzugte wasserlösliche Polymere sind Stärke und Polyvinylalkohol. Der bevorzugte Gehalt an solchen wasserlöslichen Polymeren im erfindungsgemäßen absorbierenden Polymerisat liegt bei 0-30 Gew.%, vorzugsweise 0-5 Gew%, bezogen auf die Gesamtmenge der Komponenten a) bis d). Die wasserlöslichen Polymere, vorzugsweise synthetische, wie Polyvinylalkohol, können auch als Pfropfgrundlage für die zu polymerisierenden Monomeren dienen.

Zur Initiierung der radikalischen Polymerisation werden die gebräuchlichen Initiatoren wie z.B. Azo- oder Peroxoverbindungen, Redoxsysteme oder UV-Initiatoren (Sensibilisatoren) verwendet.

Die Herstellung der erfindungsgemäßen Polymerisate erfolgt vorzugsweise nach zwei Methoden:

Nach der ersten Methode wird das teilneutralisierte Monomere a), vorzugsweise die Acrylsäure in wäßriger Lösung in Gegenwart von Vernetzern und ggf. weiteren Komponenten durch radikalische Polymerisation in ein Gel überführt, das zerkleinert, getrocknet, gemahlen und auf die gewünschte Partikelgröße abgesiebt wird. Diese Lösungspolymerisation kann kontinuierlich oder diskontinuierlich durchgerührt werden. Der Stand der Technik weist ein breites Spektrurn an Variationsmöglichkeiten hinsichtlich der Konzentrationsverhältnisse, Temperaturen, Art und Menge der Initiatoren aus. Typische Verfahren sind in den folgenden Veröffentlichungen beschrieben: US 4 286 082, DE 27 06 135 und US 4 076 663, deren entsprechende Offenbarung hiermit als Referenz eingeführt wird.

Auch die inverse Suspensions- und Emulsionspolymerisation kann zur Herstellung der erfindungsgemäßen Produkte angewendet werden. Gemäß diesen Prozessen wird eine wäßrige, teilneutralisierte Lösung der Monomeren a), vorzugsweise Acrylsäure mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenfalls während der Polymerisation zugefügt. Gegebenenfalls erfolgt die Zugabe eines wasserlöslichen Polymeren d) als Pfropfgrundlage über die Monomerlösung oder durch direkte Vorlage in die Ölphase. Anschließend wird das Wasser azeotrop aus dem Gemisch entfernt und das Polymerisat abfiltriert und ggf. getrocknet. Die Vernetzung kann durch Einpolymerisation eines in der Monomerenlösung gelösten polyfunktionellen Vernetzers und/oder durch Reaktion geeigneter Vernetzungsmittel mit funktionellen Gruppen des Polymeren während der Polymerisationsschritte erfolgen. Die Verfahren sind beispielsweise in den Veröffentlichungen US 43 40 706, DE 3713 601, DE 28 40 010 und WO 96/05234 beschrieben, deren entsprechende Offenbarung hiermit als Referenz eingeführt wird.

Die Trocknung des Polymerisatgels erfolgt bis zu einem Wassergehalt von 0,5-25 Gew.%, vorzugsweise von 1 bis 10 Gew.%, besonders bevorzugt 1 bis 8 Gew.% bei Temperaturen, die üblicherweise im Bereich von 100 - 200 °C liegen.

Hinsichtlich der Teilchenform des erfindungsgemäßen absorbierenden Polymerisaten gibt es keine besonderen Einschränkungen. Das Polymerisat kann in Form von Kügelchen vorliegen, die durch inverse Suspensionspolymerisation erhalten wurden, oder in Form von unregelmäßig geformten Teilchen, die durch Trocknung und Pulverisierung der Gelmasse aus der Lösungspolymerisation stammen. Die Teilchengröße liegt normalerweise unter 3000 µm, bevorzugt zwischen 20 und 2000 µm, und besonders bevorzugt zwischen 150 und 850 µm.

Die erfindungsgemäßen Nachvernetzerkomponenten werden in Form ihrer wässrigen Lösungen aufgebracht. Geeignete Lösungsmittel sind Wasser und ggf. polare, mit Wasser mischbare organische Lösungsmittel, die leicht feucht sind, wie beispielsweise Aceton, Methanol, Ethanol oder 2-Propanol bzw. deren Gemische. Der Begriff wäßrige Lösung im Sinne der Erfindung bedeutet in Bezug auf die Lösungsmittelkomponente, daß neben dem Wasser auch noch organische Lösungsmittel enthalten sein können. Die Konzentration der jeweiligen Nachvemetzerkomponente in dem wässrigen Lösungsmittel kann in weiten Grenzen schwanken und liegt im Bereich von 1 bis 80 Gew.%, vorzugsweise im Bereich von 5 bis 65 Gew.% und ganz besonders bevorzugt in einem Bereich von 10 bis 40 Gew.%. Das bevorzugte Lösungsmittel für die Polyole als Nachvernetzungsmittel bzw. für die Salzkomponente ist Wasser, das in einer Gesamtmenge von 0,5 - 10 Gew.%, bevorzugt 0,75 - 5 Gew.%, und besonders bevorzugt 1,0 - 4 Gew.%, bezogen auf das Polymerisat zu verwenden ist.

Eine bevorzugte Lösung besteht beispielsweise aus 1,5 - 3 Gew.Teilen Wasser, 0,5 - 1 Gew.Teilen Polyolkomponente und 0,4 - 0,6 Gew.Teilen eines anorganischen Salzes. Die gesamte Menge an Lösungsmittel wird üblicherweise im Bereich von 0,5 - 10 Gew.%, bevorzugt bei 1 - 7 Gew.% und besonders bevorzugt bei 1 - 5 Gew.% bezogen auf das Polymerisat eingesetzt.

Abhängig von der Löslichkeit der beiden Komponenten e) und f) wird die Lösung vor dem Aufbringen auf das Polymerisat auf 20-100 °C, bevorzugt auf 20-60 °C erwärmt. Ein getrenntes, vorzugsweise gleichzeitiges Zudosieren von einer wäßrigen Lösung des Polyols und einer wäßrigen Lösung der Salzkomponente ist ebenfalls möglich, wenn eine homogene Verteilung beider Komponenten auf dem Polymerisat gewährleistet ist. Bevorzugt ist das Aufbringen einer einzigen wäßrigen Lösung auf das Polymerisat, in der beide Komponenten gelöst sind.

Die Nachvernetzerlösung sollte sehr gut mit den Polymerteilchen vermischt werden. Geeignete Mischaggregate zum Aufbringen der Nachvernetzerlösung sind z.B. Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymerisat-Pulver mittels rotierender Messer in schneller Frequenz gemischt wird (Schugi-Mischer).

Es besteht auch die Möglichkeit, die Beschichtung des Polymerisates während eines Verfahrensschrittes bei der Herstellung des Polymerisates vorzunehmen. Hierzu ist besonders der Prozeß der inversen Suspensionspolymerisation geeignet.

Nachdem die Nachvernetzerlösung mit den Polymerteilchen vermischt worden ist, erfolgt die Nachvernetzungsreaktion bei Temperaturen im Bereich von > 150°C bis 250°C, bevorzugt 160°C bis 220°C und besonders bevorzugt 170°C bis 200°C. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drehrohröfen, Wirbelbetttrockner, Tellertrockner, Paddeltrockner oder Infrarottrockner genannt.

Die Polymeren gemäß der Erfindung können in großtechnischer Weise nach dem vorstehend aufgeführten, bekannten Verfahren kontinuierlich oder diskontinuierlich hergestellt werden, wobei die erfindungsgemäße Nachvernetzung entsprechend erfolgt.

Die erfindungsgemäßen Polymerisate können für weite Anwendungsgebiete eingesetzt werden.

Wenn sie z. B. als Absorbierungsmittel in Damenbinden, Windeln oder in Wundabdeckungen verwendet werden, besitzen sie die Eigenschaft, daß sie große Mengen an Menstrationsblut, Urin oder anderen Körperflüssigkeiten schnell absorbieren. Da die erfindungsgemäßen Mittel die absorbierten Flüssigkeiten auch unter Druck zurückhalten und zusätzlich in der Lage sind, im gequollenen Zustand weitere Flüssigkeit innerhalb der Konstruktion zu verteilen, werden sie besonders bevorzugt in höheren Konzentrationen, in Bezug auf das hydrophile Fasermaterial wie z.B. Fluff eingesetzt als dies bisher möglich war. Sie eignen sich auch für den Einsatz als homogene Superabsorberschicht ohne Fluffanteil innerhalb der Windelkonstruktion, wodurch besonders dünne Windeln möglich sind. Weiterhin eignen sich die Polymere zum Einsatz in Hygieneartikel (Inkontinenzprodukte) für Erwachsene.

Solche absorbierende Hygieneprodukte besitzen in der Regel einen allgemeinen Aufbau aus einer körperzugewandten flüssigkeitsdurchlässigen Abdeckung (1), einer flüssigkeitsabsorbierenden Sauglage (2) sowie einer im wesentlichen flüssigkeitsundurchlässigen, körperabgewandten Außenschicht (3). Optional finden auch weitere Konstruktionen zur schnellen Aufnahme und Verteilung von Körperflüssigkeit (4) im Saugkern Anwendung. Diese Konstruktionen werden häufig, aber nicht zwingend zwischen der körperzugewandten flüssigkeitsdurchlässigen Abdeckung (1) und der flüssigkeitsabsorbierenden Sauglage (2) eingesetzt.

Die flüssigkeitsdurchlässige Abdeckung (1) besteht in der Regel aus einem nichtgewebten, faserigen Vlies oder einer anderen porösen Konstruktion.

Als Materialien für diese Abdeckung (1) kommen z. B. synthetische Polymere wie etwa Polyvinylchlorid oder -fluorid, Polytetrafluorethylen (PTFE), Polyvinylalkohole und -Derivate, Polyacrylate, Polyamide, Polyester, Polyurethane, Polystyrol, Polysiloxane oder Polyolefine (z.B. Polyethylen (PE) oder Polypropylen (PP)) sowie natürliche Fasermaterialien sowie beliebige Kombinationen aus den vorgenannten Materialien im Sinne von Mischmaterialien oder Verbundmaterialien oder Copolymerisaten in Frage.

Die flüssigkeitsdurchlässige Abdeckung (1) hat hydrophilen Charakter. Sie kann zudem aus einer Kombination von hydrophilen und hydrophoben Bestandteilen bestehen. Bevorzugt ist in der Regel eine hydrophile Ausrüstung der flüssigkeitsdurchlässigen Abdeckung (1), um schnelle Einsickerzeiten von Körperflüssigkeit in die flüssigkeitsabsorbierende Sauglage (2) zu ermöglichen, jedoch werden auch partiell hydrophobierte Abdeckungen (1) verwendet.

Die flüssigkeitsabsorbierende Sauglage (2) enthält die superabsorbierenden Pulver bzw. Granulate und ggf. weitere Komponenten aus beispielsweise faserigen Materialien, schaumförmigen Materialien, filmbildenden Materialien oder porösen Materialien sowie Kombinationen von zwei oder mehreren dieser Materialien. Jedes dieser Materialien kann entweder natürlichen oder synthetischen Ursprungs sein oder durch chemische oder physikalische Modifikation von natürlichen Materialien hergestellt worden sein. Die Materialien können hydrophil oder hydrophob sein, wobei hydrophile Materialien bevorzugt sind. Dies gilt insbesondere für solche Zusammensetzungen, die ausgeschiedene Körperflüssigkeiten effizient aufnehmen und in Richtung zu weiter von der Eintrittsstelle der Körperflüssigkeit entfernte Regionen des absorbierenden Kerns transportieren sollen.

Als hydrophile Fasermaterialien sind geeignet z.B. Cellulosefasern, modifizierte Cellulosefasern (z.B. versteifte Cellulosefasern), Polyesterfasern (z.B. Dacron) hydrophiles Nylon oder aber auch hydrophilisierte hydrophobe Fasern, wie z. B. mit Tensiden hydrophilisierte Polyolefine (PE, PP), Polyester, Polyacrylate, Polyamide, Polystyrol, Polyurethane und andere.

Bevorzugt werden Cellulosefasern und modifizierte Cellulosefasern eingesetzt. Kombinationen von Cellulosefasern und/oder modifizierten Cellulosefasern mit synthetischen Fasern wie z. B. PE/PP Verbundmaterialien, sogenannte Bikomponentenfasern, wie sie z.B. zur Thermohondierung von Airlaidmaterialien verwendet werden oder anderen Materialien sind ebenfalls gebräuchlich.

Die Fasermaterialien können in verschiedenen Anwendungsformen vorliegen, z.B. als lose aus einem Luftstrom oder aus wässriger Phase abgeschiedene oder abgelegte Cellulosefasern, als nichtgewebtes Vlies oder als Tissue. Kombinationen verschiedener Anwendungsformen sind möglich.

Optional können neben den erfindungsgemäßen superabsorbierenden Polymerisaten weitere pulverförmige Substanzen eingesetzt werden, wie z.B. geruchsbindende Substanzen wie Cyclodextrine, Zeolithe, anorganische oder organische Salze und ähnliche Materialien.

Als poröse Materialien und schaumförmige Materialien können z.B. Polymerschäume eingesetzt werden wie sie in den Schriften DE 44 18 319 A1 und DE 195 05 709 A1 beschrieben sind.

Zur mechanischen Stabilisierung der flüssigkeitsabsorbierenden Sauglage (2) können thermoplastische Fasern (Z.B. Bikomponentenfasern aus Polyolefinen), Polyolefingranulate, Latexdispersionen oder Heisskleber verwendet werden. Optional werden eine oder mehrere Lagen Tissue zur Stabilisierung verwendet.

Die flüssigkeitsabsorbierende Sauglage (2) kann einlagig sein oder aus mehreren Schichten bestehen. Dazu können Konstruktionen verwendet, die aus hydrophilen Fasern, bevorzugt Cellulosefasern, optional einer Konstruktion zur schnellen Aufnahme und Verteilung von Körperflüssigkeit (4) wie zum Beispiel chemisch versteifte (modifizierte) Cellulosefasern oder Highloftvliese aus hydrophilen oder hydrophilisierten Fasern sowie superabsorbierenden Polymeren bestehen.

Die erfindungsgemäßen superabsorbierenden Polymerisate können dabei homogen in den Cellulosefasern oder den versteiften Cellulosefasern verteilt sein, sie können auch lagig zwischen den Cellulosefasern oder den versteiften Cellulosefasem eingebracht sein, oder die Konzentration der superabsorbierenden Polymerisate kann innerhalb der Cellulosefasern oder versteiften Cellulosefasern einen Gradienten aufweisen. Das Verhältnis der Gesamtmenge an superabsorbierendem Polymer und der Gesamtmenge an Cellulosefasern oder den versteiften Cellulosefasern im absorbierenden Saugkern kann zwischen 0 - 100 Gew.% variieren, wobei in einer Ausführungsform lokal, z. B. bei Gradienteneintrag oder schichtweisem Eintrag Konzentrationen von bis zu 100 % superabsorbierende Polymerisate möglich sind. Derartige Konstruktionen mit Bereichen hoher Konzentrationen von absorbierendem Polymerisate, wobei der Anteil des Polymerisates in bestimmten Bereichen zwischen 60 und 100 Gew.%, bevorzugt zwischen 90 und 100 Gew.% liegt, sind beispielsweise auch in der Patentschrift US 5,669,894 beschrieben.

Optional können auch mehrere verschiedene superabsorbierende Polymerisate, die sich zum Beispiel in der Sauggeschwindigkeit, der Permeabilität, der Speicherkapazität, der Absorption gegen Druck, der Komverteilung oder auch der chemischen Zusammensetzung unterscheiden gleichzeitig eingesetzt werden. Die verschiedenen Superabsorber können miteinander vermischt in das Saugkissen eingebracht werden oder aber lokal differenziert im Absorbent Core plaziert werden. Eine solche differenzierte Plazierung kann in Richtung der Dicke des Saugkissens oder der Länge oder Breite des Saugkissens erfolgen.

In der flüssigkeitsabsorbierenden Sauglage (2) befindet sich eine oder mehrere der erfindungsgemäßen, superabsorbierenden Polymerisate enthaltenden Lagen ggf. mit Cellulosefasern oder versteiften Cellulosefasern. In einer bevorzugten Ausfuhrungsfbrm werden Konstruktionen aus Kombinationen von Lagen mit homogenem Superabsorbereintrag und zusätzlich schichtweiser Einbringung verwendet.

Ggf. können die Absorptionsartikel weitere Lagen von reinen Cellulosefasern oder versteiften Cellulosefasern an der körperzugewandten Seite und / oder auch der körperabgewandten Seite aufweisen.

Die oben beschriebenen Aufbaumöglichkeiten können sich auch mehrfach wiederholen, wobei es sich um eine Aufeinanderschichtung zweier oder mehrerer gleicher Lagen oder aber auch um Aufeinanderschichtung zweier oder mehrerer unterschiedlicher Konstruktionen unterschiedlichen Aufbaus handeln kann. Dabei liegen die Unterschiede in wiederum rein konstruktiver Art oder aber im Typ des verwendeten Materials, wie z.B. die Verwendung von erfindungsgemäßen absorbierenden Polymerisaten oder mit anderen Polymerisaten, aber verschiedener Zellstoffarten.

Ggf. kann das gesamte Saugkissen oder aber auch einzelne Lagen der flüssigkeitsabsorbierenden Sauglage (2) durch Lagen von Tissue von anderen Komponenten des Absorptionsartikels getrennt oder stehen in direktem Kontakt mit anderen Lagen oder Komponenten sein.

Exemplarisch können zum Beispiel die Konstruktion zur schnellen Aufnahme und Verteilung von Körperflüssigkeit (4) und die flüssigkeitsabsorbierende Sauglage (2) durch Tissue voneinander getrennt sein oder aber in direktem Kontakt miteinander stehen. Sofern keine separate Konstruktion zur schnellen Aufnahme und Verteilung von Körperflüssigkeit (4) zwischen der flüssigkeitsabsorbierenden Sauglage (2) und der körperzugewandten flüssigkeitsdurchlässigen Abdeckung (1) existiert, sondern der Effekt der Flüssigkeitsverteilung z.B durch die Verwendung einer speziellen körperzugewandten flüssigkeitsdurchlässigen Abdeckung (1) erreicht werden soll, kann die flüssigkeitsabsorbierende Sauglage (2) ebenfalls optional von der körperzugewandten flüssigkeitsdurchlässigen Abdeckung (1) durch ein Tissue getrennt sein.

Statt Tissue kann optional auch nichtgewebtes Vlies in die flüssigkeitsabsorbierende Sauglage (2) eingebracht werden. Beide Komponenten führen zu dem erwünschten Nebeneffekt der Stabilisierung und Festigung des Absorptionskerns im feuchten Zustand.

Die flüssigkeitsabsorbierende Sauglage, insbesondere faserhaltige, superabsorbierende Polymerisate haltige, flüssigkeitsverteilende und -speichernde Schichten lassen lassen sich nach einer Vielzahl von Herstellverfahren erzeugen.

Neben den etablierten konventionellen Prozessen, wie die sich unter Drumforming mit Hilfe von Formrädern, -taschen und Produktformen und entsprechend angepassten Dosiereinrichtungen für die Rohstoffe zusammengefaßt werden können, sind moderne etablierte Verfahren wie der Airlaidprozess (z.B. EP 850 615, Sp. 4 Zeile 39 bis Sp. 5 Zeile 29, US 4.640.810) mit allen Formen der Dosierung, Ablage der Fasern und Verfestigung wie Hydrogenbonding (z.B. DE 197 50 890, Sp. 1 Zeile 45 bis Sp. 3 Zeile 50, Thermobonding, Latexbonding (z.B. EP 850 615, Sp. 8 Zeile 33 bis Sp. 9 Zeile 17 und Hybridbonding, der Wetlaid Prozeß (z.B. PCT WO 99/49905, Sp. 4 Zeile 14 bis Sp. 7 Zeile 16) , Carding-, Meltblown-, Spunblown- Prozesse sowie ähnliche Prozesse zur Herstellung von superabsorberhaltigen Non-Wovens (im Sinne der der Definition der EDANA, Brüssel) auch in Kombinationen dieser Verfahren mit- und untereinander als übliche Methoden zur Herstellung von den o.g. Flüssigkeitsspeichern zu verstehen.

Als weitere Herstellungsverfahren kommen die Herstellung von Laminaten im weitesteten Sinne sowie von extrudierten und coextrudierten, naß- und trockensowie nachträglich verfestigten Strukturen in Frage.

Eine Kombinationen dieser Verfahrensmöglichkeiten mit- und untereinander ist ebenfalls möglich.

Für die Herstellung von Absorptionsartikeln mit einer schnellen Aufnahme und Verteilung von Körperflüssigkeit (4) können zusätzlich zum Beispiel chemisch versteiften (modifizierte) Cellulosefasern oder Highloftvliesen aus hydrophilen oder hydrophilisierten Fasern oder einer Kombination von beidem mitverwendet werden.

Chemisch versteifte, modifizierte Cellulosefasern können zum Beispiel erzeugt werden aus Cellulosefasern, die durch Vernetzer wie z.B. $C_2$ - $C_8$ Dialdehyde, $C_2$ - $C_8$ Monoaldehyde mit einer zusätzlichen Säurefunktion oder $C_2$ - $C_9$ Polycarbonsäuren in einer chemischen Reaktion umgesetzt werden. Spezielle Beispiele sind: Glutaraldehyd, Glyoxal, Glyoxalsäure oder Zitronensäure. Ebenfalls bekannt sind kationisch modifizierte Stärke oder Polyamid-Epichlorhydrinharze (z. B. KYMENE 557H, Hercules Inc., Wilmington , Delaware). Durch die Vernetzung wird eine verdrehte, gekräuselte Struktur erreicht und stabilisiert, die sich vorteilhaft auf die Geschwindigkeit der Flüssigkeitsaufnahme auswirkt.

Die absorbierenden Hygieneprodukte können in ihrem Flächengewicht und Dicke und damit der Dichte stark variieren. Typischerweise liegen die Dichten der Bereiche der Absorptionskerne zwischen 0,08 und 0,25 g/cm$^3$. Die Flächengewichte liegen zwischen 10 und 1000 g/m$^2$, wobei bevorzugt Flächengewichte zwischen 100 und 600 g/m$^2$ realisiert werden (siehe auch US 5,669,894). Die Dichte variiert in der Regel über die Länge des absorbierenden Kerns. Dies tritt als Folge einer gezielten Dosierung der Cellulosefaser- oder versteiften Cellulosefasermenge oder der Menge des superabsorbierenden Polymerisats ein, da diese Komponenten in bevorzugten Ausführungsformen stärker in den Frontbereich des absorbierenden Einwegartikels eingebracht werden.

Die erfindungsgemäßen Polymeren werden auch in Absorberartikeln eingesetzt, die für weitere Verwendungen geeignet sind. Dazu werden sie durch Mischen mit Papier oder Fluff oder synthetischen Fasern oder durch Verteilen der superabsorbierenden Polymerisate zwischen Substraten aus Papier, Fluff oder nicht gewebten Textilien oder durch Verarbeitung in Trägermaterialien zu einer Bahn verarbeitet. Desweiteren finden die erfindungsgemäßen Polymeren auch überall dort Verwendung, wo wässrige Flüssigkeiten absorbiert werden müssen, wie z. B. bei Kabelummantelungen, in Lebensmittelverpackungen, im Agrarbereich bei der Pflanzenaufzucht und als Wasserspeicher sowie als Wirkstoffträger mit einer zeitlich verzögerten Freisetzung des Wirkstoffes an die Umgebung.

Die erfindungsgemäßen superabsorbierenden Polymerisate zeigen überraschenderweise eine bedeutende Verbesserung der Permeabilität, d. h. eine Verbesserung des Flüssigkeitstransportes im gequollenen Zustand. Es werden Polymerisate mit Permeabilitäts-Werten (SFC) von bis zu 70 · 10$^{-7}$ cm$^3$ s /g bei einer Retention (TB) von mindestens 27 g/g erhalten, vorzugsweise Polymere mit SFC-Werten von > 70 · 10$^{-7}$ bis ≥150 ·10$^{-7}$ cm$^3$ s /g bei einer Retention (TB) von mindestens 25 g/g. Neben diesen ausgezeichneten SFC- und Retentionswerten zeigen die erfindungsgemäßen Polymere Meßwerte für die Flüssigkeitsaufnahme unter Druck (AAP 0,7) von mindestens 18 g/g.

Die erfindungsgemäßen Produkte mit dieser hervorragenden Eigenschaftskombination aus sehr hohen SFC-Werten, hoher Retention und hoher Absorption unter Druck können ohne die Verwendung toxikologisch bedenklicher Substanzen hergestellt werden.


Testmethoden


Zur Charakterisierung der erfindungsgemäßen, absorbierenden Polymerisate werden Retention (TB), Aufnahme unter Druck (AAP) und die Durchlässigkeit für 0,9%ige Kochsalzlösung im gequollenen Zustand (SFC) bestimmt.

a) Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat läßt man als Blindwert mitlaufen.

$$\text{Retention = Auswaage-Blindwert/Einwaage [g/g]}$$

b) Flüssigkeitsaufnahme unter Druck (AAP-Test, gemäß EP 0 339 461)

Die Aufnahme unter Druck (Druckbelastung 50 g/cm$^2$) wird nach einer in der EP 0339461, Seite 7, beschriebenen Methode bestimmt. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor gewogene Zylinder wird anschließend auf eine Glasfilterplatte gestellt, die sich in einer Schale mit 0,9%iger NaCl-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

$$\text{AAP = Auswaage (Zylindereinheit + Superabsorber)-Einwaage (Zylindereinheit +}$$

$$\text{vollgesogener Superabsorber) / Einwaage Superabsorber}$$

c) Permeabilität im gequollenen Zustand (SFC-Test, gemäß WO 95/22356)

In einen Zylinder mit Siebboden werden ca. 0,9 g Superabsorbermaterial eingewogen und sorgfältig auf der Siebfläche verteilt. Das Superabsorbermaterial läßt man in JAYCO synthetischen Urin [Zusammensetzung: 2,0 g Kaliumchlorid; 2,0 g Natriumsulfat; 0,85 g Ammoniumdihydrogenphosphat; 0,15 g Ammoniumhydrogenphosphat; 0,19 g Calciumchlorid; 0,23 g Magnesiumchlorid als wasserfreie Salze in 1 l destilliertem Wasser gelöst] 1 Stunde lang gegen einen Druck von 20 g/cm$^2$ quellen. Nach Erfassung der Quellhöhe des Superabsorbers läßt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Meßtemperatur 20-25°C) während der Messung bezüglich der Gelbettbeschaffenheit gewährleistet. Der auf den gequollenen Superabsorber wirkende Druck ist weiterhin 20 g/cm$^2$. Mit Hilfe eines Computers und einer Waage wird die Flüssigkeitsmenge, die die Gelschicht als Funktion der Zeit passiert in Intervallen von 20 Sekunden innerhalb einer Zeitperiode von 10 Minuten erfaßt. Die Fließrate g/s durch die gequollene Gelschicht wird mittels Regressionsanalyse mit Extrapolation der Steigung und Ermittlung des Mittelpunkts auf den Zeitpunkt t=0 der Fließmenge innerhalb der Minuten 2-10 ermittelt. Die Berechnung des SFC-Wertes (K) berechnet sich wie folgt:

$$K = \frac{F_s(t{=}0)\cdot L_o}{r\cdot A\cdot \Delta P} = \frac{F_s(t{=}0)\cdot L_0}{139506}$$

Wobei:

$F_s$ (t = 0)    die Fließrate in g/s

$L_0$           die Dicke der Gelschicht in cm

r             die Dichte der NaCl-Lösung (1,003 g/cm$^3$)

A             die Fläche der Oberseite der Gelschicht im Meßzylinder (28,27 cm$^2$)

$\Delta P$      der hydrostatische Druck, der auf der Gelschicht lastet (4920 dyne/cm$^2$)

und

K    der SFC-Wert ist [cm$^3$ * s * g$^{-1}$]

Die formale Addition der Zahlenwerte der Teebeutelretention und des SFC-Wertes verdeutlicht den sprunghaften Anstieg dieser Eigenschaftskombination bei den erfindungsgemäßen Polymerisaten im Vergleich zu unbehandeltem Superabsorberpulver oder Produkten die nach bekannten Methoden oberflächlich nachvernetzt wurden. Der Zahlenwert wird bei den erfindungsgemäßen Produkten nicht durch einen hohen Beitrag einer der beiden Werte erreicht (z. B.eines hohen TB-Retentionswertes und eines niedrigen SFC-Wertes und umgekehrt).

Beispiele

In den Beispielen und Vergleichsbeispielen wurde das für die Oberflächenvernetzung vorgesehene Pulver jeweils auf eine Teilchengröße von 150 µm bis 850 µm abgesiebt.

Beispiel 1

In 965,115 g einer wässrigen Lösung von Natriumacrylat mit einem Neutralisationsgrad von 70 mol% (Monomer-Konzentration: 37,7 %) werden 1,05 g Polyethylenglycol(300)diacrylat und 1,35 g Polyethylenglycol-(750)monoallyletheracrylat als Vernetzer gelöst. Die Monomerenlösung wird in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natriumperoxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, wurde das Gel mit einem Fleischwolf zerkleinert und 2 h bei 150 °C in einem Umluftofen getrocknet. Das getrocknete Produkt wurde grob zerstoßen, gemahlen und die Partikel der Größe 150 - 850 µm zur weiteren Umsetzung ausgesiebt (Pulver A).
50 g Pulver A wurden unter kräftigem Rühren mit einer Lösung aus 0,5 g 1,3-Propandiol, 1,25 g Wasser und 0,25 g Aluminiumsulfat-18-Hydrat vermischt und danach für 30 min. in einem Ofen, der auf 180 °C temperiert war, erhitzt.
Zum Vergleich wurden 50 g Pulver A mit einer Lösung aus 0,5 g 1,3-Propandiol und 1,25 g Wasser vermischt und anschließend für 30 min. in einem Ofen, der auf 180 °C temperiert war, erhitzt (Vergleichsbeispiel 1)

| Produkt | TB [g/g] | AAP$_{0.7}$ [g/g] | SFC [cm$^3$ s 10$^{-7}$/g] | TB+SFC |
|---|---|---|---|---|
| **Pulver A** | 31,0 | | 0 | 31,0 |
| **Beispiel 1** | 26,0 | 22,6 | 130 | 156,0 |
| Vergleichsbeispiel 1 | 27,0 | 24,0 | 40 | 67 |

**Beispiel 2**

In 965,175 g einer wässrigen Lösung von Natriumacrylat mit einem Neutralisationsgrad von 70 mol% (Monomer-Konzentration: 37,7 %) werden 0,84 g Triallylamin und 1,5 g Polyethylenglycol (750)monoallyletheracrylat als Vernetzer gelöst. Die Monomerenlösung wird in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natriumperoxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, wurde das Gel mit einem Fleischwolf zerkleinert und 2 h bei 150 °C in einem Umluftofen getrocknet. Das getrocknete Produkt wurde grob zerstoßen, gemahlen und die Partikel der Größe 150 - 850 µm zur weiteren Umsetzung ausgesiebt (Pulver B).
50 g Pulver B wurden unter kräftigem Rühren mit einer Lösung 0,25 g Aluminiumsulfat-18-Hydrat und 0,25 g Wasser und anschließend mit einer Lösung aus 0,5 g Ethylenglycol und 0,5 g Wasser vermischt und danach für 60 min. in einem Ofen, der auf 170 °C temperiert war, erhitzt (Beispiel 2).
50 g Pulver B wurden unter kräftigem Rühren mit einer Lösung 0,5 g Glycerin, 0,05 g Ethylenglycoldiglycidylether und 1,25 g Wasser vermischt und danach für 60 min. in einem Ofen, der auf 170 °C temperiert war, erhitzt (Vergleichsbeispiel 2).
50 g Pulver B wurden unter kräftigem Rühren mit einer Lösung 0,25 g Glycerin, 0,25 g Ethylenglycoldiglycidylether und 1,25 g Wasser vermischt und danach für 60 min. in einem Ofen, der auf 170 °C temperiert war, erhitzt (Vergleichsbeispiel 3).
50 g Pulver B wurden unter kräftigem Rühren mit einer Lösung 0,25 g Ethylenglycol, 0,25 g Ethylendiamin und 1,25 g Wasser vermischt und danach für 60 min. in einem Ofen, der auf 170 °C temperiert war, erhitzt (Vergleichsbeispiel 4).

| Produkt | TB [g/g] | AAP$_{0.7}$ [g/g] | SFC [cm$^3$ s 10$^{-7}$/g] | TB+SFC |
|---|---|---|---|---|
| **Pulver B** | 30,5 | | 0 | 30,5 |
| **Beispiel 3** | 26,5 | 23,5 | 60 | 86,5 |
| **Vergleichsbeispiel 3** | 26,0 | 23,5 | 35 | 61,0 |
| **Vergleichsbeispiel 4** | 26,4 | 24,0 | 33 | 59,4 |
| **Vergleichsbeispiel 5** | 27,0 | 23,5 | 10 | 37,0 |

Industrielle Anwendbarkeit:

Die beschriebenen Beispiele des erfindungsgemäßen Verfahrens zeigen alle eine sehr gute Gesamtperformance insbesondere hinsichtlich Retention und Permeabilität. Es werden fließfähige, leicht dosierbare beschichtete Pulver erhalten. Die Verwendung kleiner Lösungsmengen zur Beschichtung, der Verzicht auf organische Lösungsmittel, anorganische Pulver oder sonstige Hilfsmittel ermöglicht ein wirtschaftliches und sicheres Produktionsverfahren. Eine deutliche Verbesserung der Permeabilität bei gleichzeitig hoher Retention ist ausschließlich durch Kombination von organischem Nachvernetzer und der Salzkomponente zu erzielen.

**Patentansprüche**

1. Pulverförmiges, an der Oberfläche nachvemetztes, Wasser, wäßrige oder seröse Flüssigkeiten sowie Blut absorbierendes Polymerisat, aufgebaut aus

   a) 55-99,9 Gew% polymerisierten, ethylenisch ungesättigten, säuregruppenenthaltenden Monomeren, die zu mindestens 25 Mol% neutralisiert sind,

   b) 0-40 Gew% polymerisierten, ethylenisch ungesättigten, mit a) copolymerisierbaren Monomeren,

   c) 0,1 - 5,4 Gew% eines oder mehrerer einpolymerisierter Vernetzer,

   d) 0-30 Gew% eines wasserlöslichen Polymeren
   wobei die Summe der Gewichtsmengen a) bis d) 100 Gew.% beträgt, **dadurch gekennzeichnet, daß** das Polymerisat mit

   e) 0,01 bis 5 Gew.%, bezogen auf das Polymerisat, wenigstens eines Polyols als Oberflächennachvernetzermittel in einer wässrigen Lösung und mit

   f) 0,001 - 1,0 Gew%, bezogen auf das Polymerisat, eines Kations in Form eines in einer wäßrigen Lösung gelösten Salzes beschichtet und auf eine Nachvernetzungstemperatur von > 150 bis 250 °C erhitzt worden ist,

   wobei das Gewichtsverhältnis von Salz zu Polyol im Bereich von 1:0,8 bis 1:4 liegt und Gesamtmenge der wäßrigen Lösungen 0,5 bis 10 Gew.%, bezogen auf das Polymerisat, betragen hat,
   mit Ausnahme von vernetzten, teilneutralisierten Polyacrylsäuren, die mit $Al_2(SO_4)_3$ und Glycerin im Gewichtsverhältnis 1:1 bzw. mit $Al_2(SO_4)_3$ ·16 $H_2O$ und Polyethylenglykol im Gewichtsverhältnis 1:1,8 bzw. mit $Al_2(SO_4)_3$ ·14 $H_2O$ zu Ethylenglykol im Gewichtsverhältnis 1:2 bzw. mit $Al_2(SO_4)_3$ ·18 $H_2O$ zu Ethylenglykol im Gewichtsverhältnis 1:2 bzw. $Al_2(SO_4)_3$ ·18 $H_2O$ zu Propylenglykol im Gewichtsverhältnis 1:1,6 behandelt worden sind.

2. Polymerisat nach Anspruch 1, **dadurch gekennzeichnet, daß** die Komponente e) mit 0,1 bis 2,5 Gew%, bevorzugt mit 0,5 bis 1,5 Gew.% und die Komponente f) mit 0,005 bis 0,5 Gew.%, bevorzugt mit 0,01 bis 0,2 Gew.% eingesetzt worden ist.

3. Polymerisat nach Anspruch 1 oder 2, **dadurch gekennzeichnet, daß** nur Wasser als Lösungsmittel für die Komponenten e) und f) eingesetzt wurde.

**4.** Polymerisat nach den Ansprüchen 1 bis 3, **dadurch gekennzeichnet, daß** die Komponenten e) und f) gemeinsam in einer wäßrigen Lösung eingesetzt wurden.

**5.** Polymerisat nach den Ansprüchen 1 bis 4, **dadurch gekennzeichnet, daß** das Polymerisat bei einer Permeabilität (SFC) bis zu 70 ·10$^{-7}$s cm$^3$ /g eine Retention (TB) von mindestens 27 g/g aufweist.

**6.** Polymerisat nach den Ansprüchen 1 bis 5, **dadurch gekennzeichnet, daß** das Polymerisat bei einer Permeabilität (SFC) von > 70 · 10$^{-7}$ bis 150·10$^{-7}$s cm$^3$ /g eine Retention (TB) von mindestens 25 g/g aufweist.

**7.** Polymerisat nach den Ansprüchen 5 oder 6, **dadurch gekennzeichnet, daß** das Polymerisat eine Flüssigkeitsaufnahme unter Druck (AAP 0,7) von mindestens 18 g/g aufweist.

**8.** Verfahren zur Herstellung von absorbierenden Polymerisaten nach den Ansprüchen 1 bis 7, **dadurch gekennzeichnet, daß** man eine Mischung aus

   a) 55-99,9 Gew% ethylenisch ungesättigten, säuregruppentragenden Monomeren, die zu mindestens 25 Mol% neutralisiert sind,

   b) 0-40 Gew% ethylenisch ungesättigten, mit a) copolymerisierbaren Monomeren,

   c) 0,1 - 5,0 Gew% eines oder mehrerer Vernetzerverbindungen,

   d) 0-30 Gew% eines wasserlöslichen Polymeren
   wobei die Summe der Komponenten a) bis d) 100 Gew.% beträgt, radikalisch polymerisiert, ggf. zerkleinert, trocknet, pulverisiert, siebt und daß man das Polymerisatpulver mit

   e) 0,01 bis 5 Gew.%, bezogen auf das Polymerisat, mindestens eines Polyols als Oberflächennachvernetzungsmittels in Form einer wässrigen Lösung
   und mit

   f) 0,001 - 1,0 Gew%, bezogen auf das Polymerisat, eines Kations eines in einer wässrigen Lösung gelösten Salzes behandelt,

wobei die Gesamtmenge Wasser 0,5 - 10 Gew.%, bezogen auf das Polymerisat beträgt, und das Gewichtsverhältnis von Salz zu Polyol im Bereich von 1:0,8 bis 1:4 liegt,
wobei eine intensive Mischung der gemeinsam oder getrennt vorliegenden, wässrigen Lösungen der Komponenten e) und f) mit dem Polymerisatpulver gleichzeitig oder nacheinander erfolgt und durch anschließendes Erhitzen auf > 150°C bis 250°C eine thermische Nachvernetzung des Polymerisatpulvers erfolgt.

**9.** Verwendung der Polymerisate nach einem der Ansprüche 1 bis 7 als Absorptionsmittel für Wasser oder wäßrige Flüssigkeiten, vorzugsweise in Konstruktionen zur Aufnahme von Körperflüssigkeiten, in geschäumten und nicht geschäumten Flächengebilden, in Verpackungsmaterialien, in Konstruktionen für die Pflanzenaufzucht als Bodenverbesserungsmittel- oder als Wirkstoff-Träger.

**10.** Verwendung der Polymerisate nach den Ansprüchen 1 bis 7 als überwiegendes oder alleiniges Absorptionsmittel in absorbierenden Einlagen.

**11.** Hygieneartikel, beinhaltend ein Polymerisat nach einem der Ansprüche 1 bis 7.

**Claims**

**1.** Pulverulent polymer which is after-crosslinked on the surface, absorbs water, aqueous or serous liquids and blood and is built up from

   a) 55-99.9 wt.% of polymerized, ethylenically unsaturated monomers which contain acid groups and are neutralized to the extent of at least 25 mol%,

b) 0-40 wt.% of polymerized, ethylenically unsaturated monomers which can be copolymerized with a),

c) 0.1-5.0 wt.% of one or more polymerized-in crosslinking agents,

d) 0-30.wt.% of a water-soluble polymer
the sum of the amounts by weight of a) to d) being 100 wt.%, **characterized in that** the polymer has been coated with

e) 0.01 to 5 wt.%, based on the polymer, of at least one polyol as an agent for after-crosslinking of the surface in an aqueous solution and with

f) 0.001 - 1.0 wt.%, based on the polymer, of a cation in the form of a salt dissolved in an aqueous solution and has been heated to an after-crosslinking temperature of >150 to 250°C,

the weight ratio of salt to polyol being in the range from 1:0.8 to 1:4 and the total amount of aqueous solutions having been 0.5 to 10 wt.%, based on the polymer,
excluding crosslinked partly neutralized polyacrylic acids which have been treated with $Al_2(SO_4)_3$ and glycerol in a weight ratio of 1:1 or with $Al_2(SO_4)_3 \cdot 16H_2O$ and polyethylene glycol in a weight ratio of 1:1.8 or with $Al_2(SO_4)_3 \cdot 14H_2O$ and ethylene glycol in a weight ratio of 1:2 or with $Al_2(SO_4)_3 \cdot 18H_2O$ and ethylene glycol in a weight ratio of 1:2 or $Al_2(SO_4)_3 \cdot 18H_2O$ and propylene glycol in a weight ratio of 1:1.6.

2. Polymer according to claim 1, **characterized in that** component e) has been employed in an amount of 0.1 to 2.5 wt.%, preferably 0.5 to 1.5 wt.%, and component f) has been employed in an amount of 0.005 to 0.5 wt.%, preferably 0.01 to 0.2 wt.%.

3. Polymer according to claims 1 or 2, **characterized in that** only water has been employed as the solvent for components e) and f).

4. Polymer according to claims 1 to 3, **characterized in that** components e) and f) have been employed together in an aqueous solution.

5. Polymer according to claims 1 to 4, **characterized in that** the polymer has a retention (TB) of at least 27 g/g at a permeability (SFC) of up to $70 \cdot 10^{-7}$s cm$^3$/g.

6. Polymer according to claims 1 to 5, **characterized in that** the polymer has a retention (TB) of at least 25 g/g at a permeability (SFC) of $>70 \cdot 10^{-7}$ to $150 \cdot 10^{-7}$s cm$^3$/g.

7. Polymer according to claims 5 or 6, **characterized in that** the polymer has an absorption of liquid under pressure (AAP 0.7) of at least 18 g/g.

8. Process for the preparation of absorbent polymers according to claims 1 to 7, **characterized in that** a mixture of

a) 55-99.9 wt.% of ethylenically unsaturated monomers which carry acid groups and are neutralized to the extent of at least 25 mol%,

b) 0-40 wt.% of ethylenically unsaturated monomers which can be copolymerized with a),

c) 0.1-5.0 wt.% of one or more crosslinking compounds,

d) 0-30 wt.% of a water-soluble polymer
the sum of components a) to d) being 100 wt.%, is subjected to free-radical polymerization and the product is optionally comminuted, dried, pulverized and sieved, and **in that** the polymer powder is treated with

e) 0.01 to 5 wt.%, based on the polymer, of at least one polyol as an agent for after-crosslinking of the surface in the form of an aqueous solution and with

f) 0.001 - 1.0 wt.%, based on the polymer, of a cation of a salt dissolved in an aqueous solution,

the total amount of water being 0.5 - 10 wt.%, based on the polymer, and the weight ratio of salt to polyol being in the range from 1:0.8 to 1:4,
wherein an intensive mixing of the aqueous solutions, present together or separately, of components e) and f) with the polymer powder is carried out simultaneously or successively and thermal after-crosslinking of the polymer powder is carried out by subsequent heating to >150°C to 250°C.

9. Use of the polymers according to one of claims 1 to 7 as absorbents for water or aqueous liquids, preferably in constructions for absorption of body fluids, in foamed and non-foamed sheet-like structures, in packaging materials, in constructions for plant growing, as soil improvement agents or as active compound carriers.

10. Use of the polymers according to claims 1 to 7 as the predominant or sole absorbent in absorbent inserts.

11. Hygienic article comprising a polymer according to one of claims 1 to 7.

**Revendications**

1. Polymère post-réticulé à la surface, en poudre, absorbant de l'eau, des liquides aqueux ou séreux ainsi que du sang, constitué de

   a) 55-99,9 % en poids de monomères polymérisés, à insaturation éthylénique contenant des groupes acides et étant neutralisés à au moins 25 % en mol,

   b) 0-40 % en poids de monomères polymérisés, à insaturation éthylénique, co-polymérisables avec a),

   c) 0,1-5,0 % en poids d'un ou de plusieurs agents de réticulation polymérisés,

   d) 0-30 % en poids d'un polymère soluble dans l'eau,
   la somme des quantités en poids a) à d) comportant 100 % en poids, **caractérisé en ce que** le polymère a été revêtu de

   e) 0,01 à 5 % en poids se référant au polymère, d'au moins un polyol en tant qu'agent de post-réticulation de la surface dans une solution aqueuse et de

   f) 0,001-1,0 % en poids se référant au polymère d'un cation en forme d'un sel dissout dans une solution aqueuse

   et a été chauffé à une température de post-réticulation de > 150 à 250°C, le rapport de poids de sel à polyol se trouvant dans l'écart de 1:0,8 à 1:4 et la quantité totale des solutions aqueuses ayant comporté 0,5 à 10 % en poids se référant au polymère,
   à l'exception d'acides polyacryliques réticulés, neutralisés partiellement qui ont été traité avec du $Al_2(SO4)_3$ et de la glycérine en rapport de poids 1:1, respectivement avec du $Al_2(SO_4)_3 \cdot 16\ H_2O$ et du polyéthylène glycol en rapport de poids 1:1,8, respectivement avec du $Al_2(SO_4)_3 \cdot 14\ H_2O$ par rapport à du polyéthylène glycol en rapport de poids 1:2, respectivement avec du $Al_2(SO_4)_3 \cdot 18\ H_2O$ par rapport à du polyéthylène glycol en rapport de poids 1:2, respectivement du $Al_2(SO_4)_3 \cdot 18\ H_2O$ par rapport à du propylène glycol en rapport de poids 1:1,6.

2. Polymère selon la revendication 1, **caractérisé en ce que** le composant e) a été utilisé avec 0,1 à 2,5 % en poids, de préférence avec 0,5 à 1,5 % en poids et le composant f) avec 0,005 à 0,5 % en poids, de préférence avec 0,01 à 0,2 % en poids.

3. Polymère selon la revendication 1 ou 2, **caractérisé en ce que** pour les composants e) et f) uniquement de l'eau a été utilisé comme solvant.

4. Polymère selon les revendications 1 à 3, **caractérisé en ce que** les composants e) et f) ont été utilisé communément dans une solution aqueuse.

5. Polymère selon les revendications 1 à 4, **caractérisé en ce qu'**avec une perméabilité (SFC) jusqu'à $70 \cdot 10^{-7}$s $cm^3$/g le polymère a une rétention (TB) d'au moins 27 g/g.

6. Polymère selon les revendications 1 à 5, **caractérisé en ce qu'**avec une perméabilité (SFC) de > 70 · $10^{-7}$ à 150 · $10^{-7}$s $cm^3$/g le polymère a une rétention (TB) d'au moins 25 g/g.

7. Polymère selon les revendications 5 ou 6, **caractérisé en ce que** le polymère a une absorption de liquide sous pression (AAP 0,7) d'au moins 18 g/g.

8. Procédé de préparation de polymères absorbants selon les revendications 1 à 7, **caractérisé en ce qu'**un mélange de

   a) 55-99,9 % en poids de monomères à insaturation éthylénique contenant des groupes acides et étant neutralisés à au moins 25 % en mol,

   b) 0-40 % en poids de monomères à insaturation éthylénique, co-polymérisables avec a),

   c) 0,1-5,0 % en poids d'un ou de plusieurs compositions d'agents de réticulation,

   d) 0-30 % en poids d'un polymère soluble dans l'eau,
   la somme des composants a) à d) comportant 100 % en poids, est soumis à une polymérisation radicalaire, est le cas échéant broyé, séché, pulvérisé, tamisé et **en ce que** la poudre de polymère est traitée avec

   e) 0,01 à 6 % en poids en se référant au polymère d'au moins un polyol en tant qu'agent de post-réticulation de la surface dans une solution aqueuse et avec

   f) 0,001-1,0 % en poids par rapport au polymère d'un cation d'un sel dissout dans une solution aqueuse,

   la quantité totale d'eau comportant 0,5 à 10 % en poids se référant au polymère et le rapport de poids de sel par rapport au polyol se trouvant dans l'écart de 1 :0,8 à 1 :4,
   un mélange intensif des solutions aqueuses étant présentes communément ou séparément des composants e) et f) avec la poudre de polymère étant effectué simultanément ou successivement et une post-réticulation thermique de la poudre de polymère étant effectuée par le chauffage subséquent à > 150°C jusqu'à 250°C.

9. Utilisation des polymères selon l'une des revendications 1 à 7 comme agent d'absorption pour de l'eau ou des liquides aqueux, de préférence dans des constructions pour absorber des liquides corporels, dans des structures superficielles moussées ou non moussées, dans des matériaux d'emballage, dans des constructions pour la culture de plantes, comme agent d'amélioration de sol ou comme porteur de composés.

10. Utilisation des polymères selon les revendications 1 à 7 comme agent d'absorption prédominant ou seul agent d'absorption dans des couches absorbantes.

11. Article d'hygiène comportant un polymère selon l'une des revendications 1 à 7.